# EUROPEAN PATENT APPLICATION

(11) **EP 4 635 393 A1**
(43) Date of publication of application: **22.10.2025**
(21) Application number: 24171267.8
(22) Date of filing: 19.04.2024
(51) Int. Cl.: A61B 1/00, G16H 40/40, G16H 50/20, A61B 5/00, A61B 90/00

(54) **SYSTEM FOR MONITORING USAGE CONDITIONS OF A MEDICAL DEVICE**

(71) Applicant: OLYMPUS Winter & Ibe GmbH, 22045 Hamburg (DE)
(72) Inventor: TIMMERMANN, Hendrik, 22045 Hamburg (DE); MÜCKNER, Andreas, 22045 Hamburg (DE); JUNGBAUER, Sebastian, 22045 Hamburg (DE)
(74) Representative: Weickmann & Weickmann PartmbB

(57) **Abstract**

The present invention relates to a system (20) for monitoring usage conditions of a medical device (10), comprising an accelerometer (22) adapted to detect acceleration of the medical device (10) during handling thereof, a further sensor (24) unit adapted to detect a further condition of the medical device (10) during handling thereof, and a control unit (26) with a dedicated storage unit (26a), wherein the control unit (26) is operatively connected to the accelerometer (22) and the further sensor unit (24) and adapted to derive use data of the medical device (10) from the output data of the accelerometer (22) and the further sensor unit (24) and to differentiate based on the respective output data of the accelerometer (22) and the further sensor unit (24) between a regular use state and an unintended use state of the medical device (10). The invention further relates to a medical device comprising such a system and a method for operating such a system or device.

## Description

The present invention relates to a system for monitoring usage conditions of a medical device, a medical device comprising such a system and a method for operating such a medical device.

In order to be able to ensure proper operation of medical equipment and devices, there is the need to perform inspection of said devices on a regular basis since any malfunctioning thereof may cause serious failures during critical therapeutical or surgical interventions at a patient. However, currently the inspection of medical equipment and devices is mainly performed by visual inspection for identifying outer damage thereto or disassembly to identify internal damage after such internal damage has been recognized due to the device not working as expected while in use.

Therefore, no predictive techniques have been used which would allow for identifying any expectable or already occurring damages to medical equipment before putting it into use such that any failures or unexpected behaviour during medical procedures could be avoided beforehand. Additionally, by only being able to identify possible damages to medical equipment by means of dissembling the corresponding device, high costs and extended downtimes of the equipment are to be expected which result in higher operational costs and general inconvenience for the users of said devices.

In particular, while outer damage on medical devices such as endoscopes or hand instruments can be identified by visual inspection by trained professionals, internal defects due to physical stress may only be identified after disassembly or through side effects such as image loss or other functionalities thereof not working properly as expected.

It is therefore the object of the present invention to be able to identify possible sources of damages to medical devices by monitoring corresponding usage conditions and to record suitable data for predictive diagnosis.

For this purpose, the present invention proposes a system for monitoring usage conditions of a medical device, comprising an accelerometer adapted to detect acceleration of the medical device during handling thereof, a further sensor unit adapted to detect a further condition of the medical device during handling thereof, and a control unit with a dedicated storage unit, wherein the control unit is operatively connected to the accelerometer and the further sensor unit and adapted to derive use data of the medical device from output data of the accelerometer and the further sensor unit and to differentiate based on the respective output data of the accelerometer and the further sensor unit between a regular use state and an unintended use state of the medical device.

Thus, the present invention provides a system by means of which two different sensor units are used in order to monitor usage conditions of a medical device of interest, wherein the control unit based on the provided data is able to differentiate whether the corresponding medical device is used in an intended way or whether events are occurring which put the medical device in a state in which damage thereto has to be expected or at least suspected. In particular, by using an accelerometer as the primary sensor unit, typical usage patterns can be detected and recorded which are expected during proper handling or operations of the device, for example while performing medical procedures or other expected situations, such as storing, sterilizing and transporting said device, while on the other hand unexpected events with excessive acceleration values, such as shocks or drops thereof with potentially harmful impact parameters may be detected as well. By adding the further sensor unit, a classification, refinement, verification and validation of the data provided by the accelerometer may be performed and the data provided by the accelerometer as well as the further sensor unit may be processed by the control unit in a advantageous way as will further be discussed in the following.

In one possible embodiment, the further sensor unit may also be formed by a second accelerometer, which may be of a different type than the first accelerometer, preferably may have a larger measurement range, in particular up to at least 200g. While such accelerometers with an extended measurement range have become available in recent times, nevertheless their power consumption is significantly increased as compared to regular accelerometers which have been optimized for low poor consumption in their reduced measurement range.

In alternative embodiments, different types of sensors may be employed as the further sensor unit. Possible types of sensors include microphones for detecting sound waves, piezo elements for detecting pressure and temperature sensors. All of said types of sensors are able to directly or indirectly detect impacts of or on the medical device in question which may be classified as unintended use states in the sense of the present invention.

To further optimize the power consumption of the corresponding system according to the present invention, the further sensor unit and in particular the second accelerometer may be adapted to normally be in a low-power stand-by mode and to transition to a functional mode only upon detection of a threshold value. Said threshold value may relate to a value of the further condition which the further sensor unit itself is monitoring or to a threshold acceleration. Therefore, in embodiments which employ a second accelerometer, said second accelerometer may be switched from the low-power stand-by mode to its functional mode only in cases that a certain acceleration is exceeded, wherein the corresponding signal may be provided by the second accelerometer itself, if it is capable of transitioning from its low-power stand-by mode to its functional mode by itself. In such embodiments, during the stand-by mode acceleration data is recorded at a lower frequency or only concerning whether said threshold value has been reached, and as soon as the threshold has been reached, highly granular and precise acceleration data is taken and recorded in the functional mode. On the other hand, the second accelerometer may also in other embodiments be transitioned to its functional mode upon receiving an activation signal from outside, for example from the first accelerometer or the control unit, as soon as it has been detected that the threshold acceleration value has been reached. In a similar manner, the first accelerometer may also be provided with a low-power mode, wherein upon a detection of a small movement amount, it transitions into its fully functional mode for providing suitable data to the control unit.

While the second accelerometer may in particular have a measurement range of up to 200g, the first accelerometer in contrast have a measurement range of up to at least 16g, preferably up to at least 32g. Such accelerometers are widely available on the market and have a low power consumption such that they are for example also used in mobile phones and smart watches for tracking usual movements of their wearers or users, respectively.

While different approaches may be used for differentiating between the regular use state and the unintended use state, in certain embodiments, the control unit may be adapted to differentiate between said two use states based on machine learning data previously recorded in the storage unit of the control unit. For example, previous to shipping the system according to the invention, extensive experiments may be performed with corresponding medical devices comprising such systems in which they are exposed to both regular use states and unintended use states wherein the collected data is then used as training data for artificial intelligence machine learning systems, such as for example neural networks.

The system according to the present invention may further comprise a communication unit, in particular a wireless communication unit, operatively coupled to the control unit. By providing said communication unit, data recorded by the accelerometer and the further sensor as well as data processed by the control unit may be transmitted to an external data processing system either in real time, intermittently or upon demand by a user in order to be able to externally perform diagnostics on the past use of the corresponding medical device.

Additionally or alternatively, the control unit may further be adapted to record use data in the storage unit for later readout, such that for example during regular inspections of the corresponding medical device, the recorded use data may be read out in order to examine past usage conditions of the medical device and to perform diagnostics.

The control unit may also be adapted to employ artificial intelligence for differentiating between the regular use state and the unintended use state of the medical device, such as for example a neural network to which the corresponding data provided by the accelerometer as well as the further sensor unit serve as input.

According to a further aspect, the present invention relates to a medical device, in particular a reprocessable medical device, comprising a system according to the invention as just described. In this context, reprocessable medical devices are understood to be multi-use medical devices, which may for example be treated in autoclaves for sterilisation between uses. On the other hand, systems according to the present invention may of course also be used in single-use devices, for example for ensuring their proper handling prior to their medical use during manufacture and/or transport thereof.

The corresponding medical device may further comprise a standalone power source, in particular a rechargeable power source, in order to be able to monitor the usage and handling thereof also during periods in which no external power source is readily connected thereto. Thus, for example in the case of an endoscope, while during surgical procedures said endoscope is connected to an external power source which may also be used to power the sensor units as well as the control unit of the above described system, by providing an additional standalone power source in the medical device, the proper handling of the device may also be monitored during times in which the device is transported, sterilized or otherwise handled without access to an external power supply.

As already briefly mentioned, while the present invention may be incorporated in any conceivable type of medical device, the invention may in particular relate to an endoscope or a case for an endoscope. Thus, it shall be understood that medical devices in the sense of the present invention not only relate to surgical equipment or similar devices directly in contact with a patient, but also to auxiliary equipment which is used for handling and transporting such medical devices, such as the above mentioned case or different types of auxiliary instruments. By monitoring the use states of such auxiliary equipment, possible damage to a device transported therein or used in combination therewith may readily be detected.

According to yet another aspect, the present invention relates to a method for operating a system or a medical device as just described, comprising continuously monitoring the acceleration of the medical device by means of the accelerometer, at least temporarily monitoring the further condition of the medical device by means of the further sensor unit, and by means of the control unit, deriving use data of the medical device from the output data of the accelerometer and the further sensor unit and differentiating based on the respective output data of the accelerometer and the further sensor unit between the regular use state and unintended use state of the medical device.

Said method may further comprise initially and/or repeatedly performing machine learning based on effectuating both regular and unintended use states with the medical device or a different medical device of the same type prior to using said medical device in its intended way and context.

One example of machine learning techniques that can be used in this context may comprise detecting a connection state of the medical device to an external device, for example a video processor, and based on the detected connection state, evaluating movement patterns of the medical device. This approach is generally based on using various thresholds in different use cases of the device, and in the particular example of disconnection thereof from the video processor, it may for example be moved from an operating room to a cleaning department, during which different movement patterns are expected compared to the use of the device during a medical procedure. By calculating the corresponding duration and measuring the movement pattern, the next steps, such as for example manual cleaning, can further be differentiated. Through the use of artificial intelligence, the understanding of this use phase of the device can be improved/learned over time. This allows for distinguishing typical durations for each identified use phase, based on which threshold settings may be adjusted to enhance system sensitivity or vice versa. Such processes and learning at each typical use step of the corresponding device contribute to energy conservation by preventing unnecessary device activation, thereby potentially extending battery life.

Lastly, the differentiating between the regular use state and the unintended use state of the device may comprise classifying impacts of the medical device. For this purpose, corresponding medical devices may be dropped from predetermined heights or may be subjected to impacts in another manner which can be classified as acceptable or as unacceptable depending on the structural integrity of the device and its general resilience. Based on the recorded experimental data, acceptable impacts can be classified as regular usage states, while excessively hard impacts such as drops from excessively large heights can be classified as unintended use states for further use during operation of the system/device.

Further features and advantages of the present invention will become even clearer from the following description of an embodiment thereof, when viewed together with the accompanying drawings. These drawings show in particular:
Fig. 1 a schematic view of a medical device according to the present invention;
Fig. 2 a schematic view of a method for impact classification performed with the device of Fig. 1; and
Fig. 3 a flow chart of a method for using the device of Fig. 1 according to the present invention.

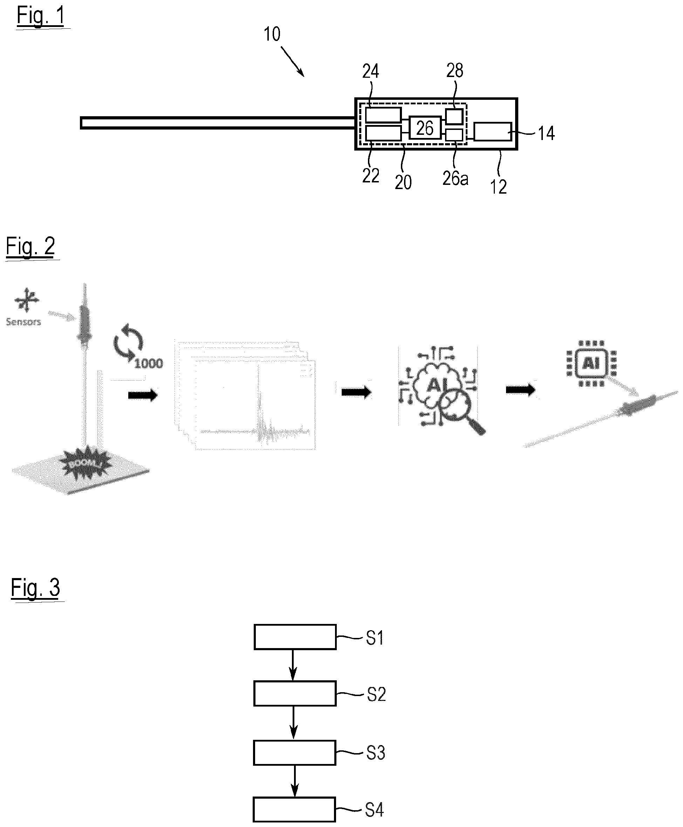

In Fig. 1, a medical device in form of an endoscope is shown schematically and generally denoted with reference numeral 10. Said endoscope comprises a housing 12 and typical functional components such as an image sensor, an optical lens, a light guide cable receiving light from an external light source, etc., which are known to the person skilled in the art and are neither shown in Fig. 1 nor explained in detail in the following.

In the housing 12 of the endoscope 10, a system 20 according to the present invention is housed as well as a standalone power supply 14, such as a rechargeable energy storage element, in particular in form of a rechargeable battery, and/or an energy harvesting unit, for example of an electromechanical type. Said system 20 comprises an accelerometer 22 for detecting acceleration of the endoscope 10 during handling thereof, a further sensor unit formed by a second accelerometer 24 with a larger measurement range compared to the first accelerometer 22 and a control unit 26 with a dedicated storage unit 26a, wherein the control unit 26 is operatively coupled to the two accelerometers 22, 24 and adapted to process the corresponding sensor data provided by the accelerometers 22 and 24.

Now referring to Fig. 2, in a schematic manner, a process for classifying impacts with the endoscope of Fig. 1 is shown. Therein, the endoscope 10 is dropped from different predetermined heights and under predetermined angles or orientations and the corresponding sensor data provided by the accelerometers 22 and 24 during fall and impact is recorded for further analysis.

Said experiment is performed many times over and the resulting detected profiles of the acceleration representing the impacts of the endoscope are fed into a suitable machine learning based algorithm for defining acceptable and unacceptable impacts on the endoscope. Based on said machine learning, the control unit 26 of the endoscope 10 is provided with suitable data and algorithms for monitoring the endoscope 10 during its use and handling with respect to whether any unexpectedly hard impacts are occurring. The corresponding data derived by the control unit 26 based on the data provided by the accelerometers 22, 24 can then be saved in the storage unit 26a for later readout and/or may be transferred periodically, in real time or based on user requests to an external server by means of a wired or wireless communication means 28 also provided in the system 20 inside the endoscope housing 12.

Fig. 3 now shows a flow chart of a method according to the present invention for operating the medical device in form of the endoscope 10 of Fig. 1. First of all, in step S1 prior to shipping and operating the endoscope 10, machine learning is performed based on subjecting the endoscope 10 to both regular and unintended use states, wherein of course a different endoscope of the same type or similar type may be used for the machine learning experimentation. Said step S1 may for example be performed by means of the method illustrated in Fig. 2 and explained above.

At a certain time during the life span of the endoscope 10, for example when said endoscope 10 leaves its factory and is being shipped to a customer, the acceleration of the medical device is started to be monitored by means of the accelerometer 22 in step S2, wherein said monitoring is continuously performed in order to detect any mishandling of the device 10 in different scenarios.

At least temporarily, a further condition of the medical device and in the embodiment explicitly explained here, a larger measurement range of the acceleration is monitored by means of the second accelerometer, for example upon detection of an exceeding of a threshold acceleration value which activates the second accelerometer for extending the measurement range concerning the acceleration of the device 10.

Based on the data provided by the two sensor units 22 and 24 in step S3, in step S4, the control unit 26 differentiates between a regular use state and an unintended use state of the medical device 10 and transmits and/or stores corresponding data for further use. Based on said collected and characterized data, predictive maintenance of the device may be performed and warnings or alarms may be output during any suitable time when a mishandling of the device is detected as an unintended state of use.

## Claims

1. System (20) for monitoring usage conditions of a medical device (10), comprising:
- an accelerometer (22) adapted to detect acceleration of the medical device (10) during handling thereof;
- a further sensor (24) unit adapted to detect a further condition of the medical device (10) during handling thereof; and
- a control unit (26) with a dedicated storage unit (26a),
wherein the control unit (26) is operatively connected to the accelerometer (22) and the further sensor unit (24) and adapted to derive use data of the medical device (10) from the output data of the accelerometer (22) and the further sensor unit (24) and to differentiate based on the respective output data of the accelerometer (22) and the further sensor unit (24) between a regular use state and an unintended use state of the medical device (10).

2. System (20) according to claim 1,
wherein the further sensor (24) unit is formed by a second accelerometer (24),wherein the second accelerometer (24) is preferably of a different type than the accelerometer (22) and/or has a larger measurement range, in particular up to at least 200g.

3. System (20) according to any of claims 1 and 2,
wherein the second accelerometer (24) is adapted to normally be in a low-power stand-by mode and to transition to a functional mode upon detection of a threshold value.

4. System (20) according to any of the preceding claims,
wherein the accelerometer (22) has a measurement range of up to at least 16g, preferably up to at least 32g.

5. System (20) according to any of the preceding claims,
wherein the control unit (26) is adapted to differentiate between the regular use state and the unintended use state based on machine learning data previously recorded in the storage unit (26a) of the control unit (26).

6. System (20) according to any of the preceding claims,
further comprising a communication unit (28), in particular a wireless communication unit, operatively coupled to the control unit (26).

7. System (20) according to any of the preceding claims,
wherein the control unit (26) is further adapted to record use data in the storage unit (26a) for later readout.

8. System (20) according to any of the preceding claims,
wherein the control unit (26) is adapted to employ artificial intelligence for differentiating between the regular use state and the unintended use state of the medical device (10).

9. Medical device (10), in particular reprocessable medical device, comprising a system (20) according to any of the preceding claims.

10. Medical device (10) according to the preceding claim,
further comprising a stand-alone power source (14), in particular a rechargeable power source.

11. Medical device (10) according to any of claims 9 and 10,
wherein the medical device (10) is an endoscope (10) or a case for an endoscope.

12. Method for operating a system (20) according to any of claims 1 to 8 or a medical device (10) according to any of claims 9 to 11,
comprising:
- continuously monitoring the acceleration of the medical device (10) by means of the accelerometer (22);
- at least temporarily monitoring the further condition of the medical device (10) by means of the further sensor unit (24);
- by means of the control unit (26), deriving use data of the medical device (10) from the output data of the accelerometer (22) and the further sensor unit (24) and differentiating based on the respective output data of the accelerometer (22) and the further sensor unit (24) between a regular use state and an unintended use state of the medical device (10).

13. Method according to the preceding claim,
further comprising:
- initially and/or repeatedly performing machine learning based on effectuating both regular and unintended use states with the medical device (10) or a different medical device of the same type.

14. Method according to any of claims 12 to 13,
further comprising:
as part of the machine learning, detecting a connection state of the medical device (10) to an external device, for example a video processor, and based on the detected connection state, evaluating movement patterns of the medical device (10).

15. Method according to any of claims 13 to 13,
wherein the differentiating between the regular use state and the unintended use state comprises classifying impacts of the medical device (10).
